Europäisches Patentamt

European Patent Office

Office européen des brevets

(11) Veröffentlichungsnummer: **0 389 996**
A1

## EUROPÄISCHE PATENTANMELDUNG

(21) Anmeldenummer: 90105607.7

(22) Anmeldetag: 24.03.90

(51) Int. Cl.5 **A61B 5/103**

(30) Priorität: 28.03.89 DE 3910018
28.03.89 DE 8903827 U

(43) Veröffentlichungstag der Anmeldung:
03.10.90 Patentblatt 90/40

(84) Benannte Vertragsstaaten:
CH DE FR GB LI NL

(71) Anmelder: **Gaddum, Friedbert**
**Schulstrasse 6**
**D-3031 Ahlden(DE)**

(72) Erfinder: **Gaddum, Friedbert, Dipl.-Ing.-Phys.**
**Schulstrasse 6**
**D-3031 Ahlden(DE)**
Erfinder: **Gabriel, Elmar, Dr. Dr. habil.**
**Dipl.-Phys.**
**Hofellernstrasse 22**
**D-8707 Veitshöchheim(DE)**

(54) Verfahren und Vorrichtung zur Messung und Beeinflussung eines lebenden Systems.

(57) Bei der statischen und/oder dynamischen Verformung von Körpern und räumlichen Gebilden aus verschiedenen in sich homogenen Werkstoffen durch von außen einwirkende mechanische Kräfte wird die zugeführte mechanische Energie zum einen Teil elastisch reversibel gespeichert, zum anderen Teil irreversibel absorbiert oder aber auch nach außen abgegeben. Diese Arten der Energieumwandlung sind material- und strukturabhängig bzw. konstruktionsabhängig zeitlich und räumlich verteilt.

Die Schüttelvorrichtung dient zur definierten Einleitung von Kräften und zur Messung entstehender Verformungen, so daß aus dem zeitlichen Verlauf der Kraftwirkungen und der Verformungen durch Berechnungsverfahren die gespeicherten und die absorbierten Energiebeträge bestimmt werden können. Wesentlich ist, daß die Orte der Krafteinleitung und der Verformungsmessung variiert werden können. Damit lassen sich aus aufeinanderfolgenden Messungen mit variierten Bedingungen Angaben machen über die lokale Verteilung der Energiespeicherung und der Energieabsorption.

Die Schüttelvorrichtung dient zur Untersuchung und mechanischen Beeinflussung vorwiegend des lebenden menschlichen Körpers, aber auch von Bauwerksmodellen, Bauteilen und jeder Art von statisch und dynamisch beanspruchten komplexen mechanischen Gebilden.

EP 0 389 996 A1

Zeichnung 1

Dynamisch – mechanisches Spektrometer
Modell EPLEXOR

Abb. A

Abb. B
Temperier – und
Kaeltekammer

Abb. C

2

## Verfahren und Vorrichtung zur Messung und Beeinflussung von Eigenschaften eines lebenden Systems

Die Erfindung besteht aus einem Verfahren und einer Vorrichtung zur Messung und Beeinflussung von Eigenschaften eines lebenden Systems, wobei nach der Methode der dynamisch-mechanischen Spektrometrie das lebende System mit konstanten Frequenzen dynamisch, aber auch statisch belastet und entlastet wird und die jeweils bei der entsprechenden Belastung oder Entlastung und Umweltsimulierung auftretenden Eigenschaftsänderungen über Kraftmeß- und Verformungsmeßsysteme kontinuierlich verfolgt werden, wobei während oder nach der Messung die zwischen den Kräften und Verformungen auftretenden zeitlichen Phasenverschiebungen mit erfaßt und ausgewertet werden.

Das lebende System kann gemäß DIN 53 513, ISO 2856. ISO 4664, aber auch gemäß der BRD Patentschrift 25 22 362 beansprucht und während der Beanspruchung messend die sich ändernden Eigenschaften des lebenden Systems verfolgen.

Hierzu kann zur Auswertung der Kraft- und Verformungsmessungen an dem lebenden System eine Fourier-, eine Hysterese- oder eine Relaxationsanalyse nach bekannten Verfahren herangezogen werden. Bisher wurden lebende Systeme mit Röntgenstrahlen, Ultraschallschwingungen und anderen Prüfmethoden untersucht, die alle nicht in der Lage waren, die zeitliche Phasenverschiebung zwischen den im Körper auftretenden Kräften und Verformungen während der Beanspruchung am lebenden System im geschlossenen Kraftfluß messend zu verfolgen.

Die Erfindung ermöglicht erstmals im kraftschlüssigen Betrieb derartige Untersuchungen an lebenden Systemen, wobei diese lebenden Systeme über die Rückkopplung das Belastungssystem beeinflussen können.

Die Erfindung ermöglicht die Untersuchung von lebenden pflanzlichen Systemen, aber auch tierischen und menschlichen lebenden Systemen.

Die Erfindung ermöglicht beispielhaft am lebenden System des Menschen, neben der Messung und Beeinflussung von Eigenschaften, die Erfassung von Vorgängen im Menschen, die auf die Reaktion der verschiedenen Organe durch die erzwungenen dynamisch-mechanischen Beanspruchungen hervorgerufen werden.

Beispielhaft dargestellt kann der Mensch als riesiges Makromolekül betrachtet werden, wobei die einzelnen Organe mit den freien Drehbarkeiten von z.B. Kunststoff- und Kautschukmolekülen verglichen werden können.

Die Erfindung ermöglicht die Veränderung der Frequenzen der dynamischen Beanspruchung des lebenden Systems. Bei einer frequenzdurchlaufenden Beanspruchung werden die verschiedenen Organe im lebenden System Resonanzen durchlaufen, die sich durch Absorptionsgebiete, d.h. maxima der Phasenverschiebung zwischen Kraft und Verformung zu erkennen geben.

Umgekehrt ist es möglich, verschiedene Organe in Resonanz zu bringen und hierdurch chemische und organische Beeinflussungen durch die dynamische Beanspruchung herbeizuführen.

Die Erfindung ermöglicht die Abnahme von Potentialen an verschiedenen Stellen am lebenden System während der dynamischen Beanspruchung und gibt daher Aufschluß über die Reaktion der Kräfte im lebenden System.

Die Erfindung bezieht sich auf eine mechanische Gestellanordnung mit einem Kraftgeber und einem Wegmeßgerät, mit welcher geometrisch definierte Objekte mechanischen Krafteinwirkungen und Verformungen ausgesetzt werden können. Die Anordnung ist vorwiegend für die Anwendung im Hinblick auf den lebenden menschlichen Körper vorgesehen, sie ist aber auch für die Beeinflussung und Untersuchung von Tieren, Pflanzen, Werkstoffstücken, Bauteilen (z. B. von Maschinen und Fahrzeugen) sowie von Modellen von Bauwerken vorgesehen.

Die Untersuchung des Verhaltens von Objekten gegenüber Krafteinwirkungen, die zu Erschütterungen, Vibrationen und damit verbundenen Deformationen führen, ist eine der wichtigsten Maßnahmen in der Technik, um die Funktionsfähigkeit technischer Objekte und ihre Widerstandsfähigkeit gegenüber Störungen und Zerstörungen durch mechanische Umwelteinflüsse zu untersuchen. Es gibt daher zahlreiche Meßanordnungen und genormte Meß vorschriften, mit denen jeweils auf mehr und weniger eng begrenzten Gebieten der Technik solche Schwingungs- und Vibrationsprüfungen mit einbezogen werden, denn auch der menschliche Körper kann bei der Beeinflussung durch mechanische Schwinungen und Vibrationen Schäden erleiden.

Mit den bisher bekannten Anordnungen ist es möglich, solchen Objekten durch definiert erzeugte Bewegungen bzw. Krafteinleitungen mechanische Energie zuzuführen, wobei sie sich je nach ihren mechanischen Eigenschaf ten mehr oder weniger verformen. Bei solchen mechanischen Beeinflussungen wird die zugeführte Energie teils reversibel gespeichert, teils irreversibel absorbiert. Diese Vorgänge können bei Werkstoffen aus solchen kraftabhängigen und frequenzabhängigen Verformungen durch Bestimmung des komplexen Elastizitätsmoduls an genormten Probestücken qualitativ und quantitativ erfaßt werden.

Bei solchen Untersuchungen wird davon ausgegangen, daß die Kraftverteilung und auch die Energiespeicherung sowie die Energieabsorption (Dämpfung) in der Probe weitgehend homogen verteilt ist oder zumindest nur geringfügig oder jedenfalls überschaubar variiert. Das ist nicht mehr der Fall bei geometrisch komplexen Systemen, wie sie z. B. das Modell einer Brückenkonstruktion oder das Skelettsystem des Menschen darstellen. In solchen Fällen ist es wichtig zu wissen, wie in solchen Strukturen die Bereiche hoher und niedriger Energieabsorption (Dämpfung) und reversibler Energiespeicherung (rein elastisches Verhalten) verteilt sind, denn aus solchen Kenntnissen lassen sich Rückschlüsse ziehen auf das Langzeitfestigkeitsverhalten bzw. auf die Risikobereiche des Systems im Hinblick auf ein mögliches Versagen. Solche Bestimmungen können mit den bisher bekannten Schüttel- bzw. Vibrationsprüfeinrichtungen quantitativ nicht und qualitativ nur beschränkt und unter speziellen Bedingungen durchgeführt werden (in Sonderfällen bei hoher Belastung, z. B. durch Infrarotemission).

Diesem Mangel will die beschriebene erfindungsgemäße Schüttelvorrichtung abhelfen. Mit ihm ist es möglich, in geometrisch einfache, aber auch in geometrisch komplex aufgebauten Objekten bzw. Strukturen statische und dynamische Kräfte einzuleiten und die Verformungsreaktion zeitabhängig und in ihrer Relation zur Krafteinleitung zu messen. Dabei können der Bereich oder können die Bereiche der Krafteinleitung ebenso wie der Bereich oder die Bereiche der Verformung und der Kraftableitung durch die definiert variierbare Art und den Ort der Befestigung des Objekts auf dem Gestell wählbar verändert werden. Auf diese Weise kann durch die variable Änderung des Kraftflusses durch das Objekt das Zusammenwirken verschiedener Objektbereiche bei der Kraftübertragung, der reversiblen Energiespeicherung und bei der Energieabsorption bestimmt werden. Aus solchen Messungen mit variierender Befestigung des Objekts lassen sich auch Rückschlüsse ziehen auf das Verhalten einzelner Objektbereiche bei mechanischer Belastung. Dies ist eine allgemein wichtige Fragestellung, die beim Menschen besondere Wichtigkeit erhält bei der Untersuchung der Funktionsfähigkeit und des Gesundheitszustandes des Skelettsystems, insbesondere der Gelenke. Erreicht wird die Gewinnung solcher Erkenntnisse durch die Untersuchung der Objekte auf der Schüttelvorrichtung, wobei für die Krafterzeugung und die Bewegungsmessung mit anschließender Auswertung diejenigen Vorrichtungen und Methoden in z. T. modifizierter Form eingesetzt werden, die in der Werkstoffprüfung als Normverfahren üblich sind und die aufgrund bekannter Schutzrechte als Stand der Technik verfügbar sind.

## Abb. 1

Zeigt ein dynamisch-mechanisches Spektrometer, das üblicherweise für die Durchführung von dynamisch-mechanischen Messungen zur Anwendung kommt. Die Erfindung bezieht sich neben der meßtechnischen Kombination auf die Abbildung B dieser Darstellung.

Die dort beschriebene Temperier- Kältekammer wird erfindungsgemäß durch eine neuartige Meßkammer ersetzt, wobei diese Kammer dann in Kombination mit dem dynamisch-mechanischen Spektrometer das Verfahren und die Vorrichtung zur Messung und Beeinflussung von Eigenschaften eines lebenden Systems darstellt.

## Abb. 2

Das der Erfindung zugrundeliegende neue Meßverfahren, einschließlich Vorrichtung, beinhaltet als 1. Beispiel eine Temperier-, Kälte- und Feuchtekammer, in der die verschiedensten Umweltverhältnisse eingestellt werden können. Diese Kammer kann zusätzlich begast und entgast werden, so daß mit verschiedenen Luftmischungen, einschließlich Sauerstoff, gearbeitet werden kann.

Die sich im Kraftfluß des dynamisch-mechanischen Spektrometers befindliche Vorrichtung verfügt über mehrere Durchführungen, von denen mindestens zwei thermisch und elektrisch isoliert ausgeführt sind. Diese beiden Durchführungen dienen zur Herstellung des Kraftflusses durch das zu untersuchende lebende System. Die Vorrichtung ist so ausgeführt, daß sie sich mitsamt der zugehörigen mechanischen Vorrichtungen, die für die Erzeugung des Kraftdurchflusses erforderlich sind, in jede beliebige Lage bringen läßt.

Das natürliche Gewicht des lebenden Systems kann durch Einbringung von Flüssigkeiten oder anderen Vorrichtungen in die Kammer beliebig verändert werden. Die Vorrichtung sorgt dafür, daß das lebende System optimal im Kraftfluß eingebettet ist. Hierzu befindet sich an der einen Seite innerhalb des Kraftflusses eine Haube, die mit einigen elektronischen Zuführungen be stückt ist. Auf der anderen Seite innerhalb des Kraftflusses befindet sich eine Mulde, die zur Aufnahme der anderen Seite des lebenden Systems zur Anwendung kommt, wobei sich auch an dieser Stelle einige Elektroden zur Abnahme von Spannungen und Zuführung von Spannungen befinden. Fußmulde und Haube können auf verschiedene Distanzen eingestellt werden. Oberhalb der Mulde befinden sich vertikale Führungen, die reibungsfrei das lebende System abstützen können. Über die kraftschlüssigen Durchführungen werden die dynamischen Verformungen eingeleitet. Auf der anderen Seite werden mit Kraftmeßsystemen die im lebenden System auftretenden Kräfte registriert.

## Zeichnung 1, 2 und 3

In den Zeichnungen 1, 2 und 3 ist der Erfindungsgegenstand in einem weiteren möglichen Ausführungs- und Anwendungsbeispiel (Mensch als Untersuchungsobjekt) in der Aufsicht sowie in frontaler und lateraler Seitenansicht dargestellt. Die Schüttelvorrichtung besteht aus einer unbeweglichen Gestellunterlage (1) mit hochklappbarer flacher Basisplatte (2). Auf dieser Platte ist ein Bewegungserzeuger (Kraftgeber) (3) angebracht, der über ein Schubstangensystem (4) Kräfte auf Bewegungsgestellteile (5-9) übertragen kann. Diese Bewegungsgestellteile sind auf der Basisplatte in Führungsböcken (10-19) verschiebbar, aber fixierbar angeordnet. Die Verbindung zwischen je 2 Führungsböcken und einem zugeordneten Bewegungsgestellteil ist über 2 auf den Führungsböcken angebrachten möglichst reibungsfreien Lagerungen (20-29) hergestellt. Diese Lagerungen können durch Justiervorrichtungen (30-39) individuell justiert werden. Die Bewegungsgestellteile können mit dem Schubstangensystem (4) durch Fixiervorrichtungen (40-49) starr verbunden werden, sie werden dadurch zu Bewegungserzeugergestellteilen, die auf ein Objekt, welches mit ihnen verbunden ist, Kräfte übertragen und damit Bewegungen in Richtung der Schubstangenlängsachsen bewirken können.

Die Anwendung der Schüttelvorrichtung sieht nun vor, daß nicht alle der (im vorliegenden Beispiel fünf) vorhandenen Bewegungsgestellteile mit dem Schubstangensystem (4) starr verbunden werden. Die nicht mit dem Schubstangensystem (4) verbundenen Gestellteile werden über ein zweites Schubstangensystem (50) mit den restlichen Bewegungsgestellteilen verbunden, die dadurch zu Bewegungsempfängergestellteilen werden. Die vom Bewegungserzeugergestellteile-Schubstangensystem in das Objekt eingeleitete Kraft wird durch das auf den Gestellteilen fixierte Objekt auf die Bewegungsempfängergestellteile und das damit verbundene Schubstangensystem (50) übertragen. Die damit relativ zur Basisplatte erzeugte Bewegung wird an den Bewegungsempfänger (51) weitergeleitet. Die Herstellung eines kraftschlüssigen Verbundes zwischen dem Schubstangensystem (50) und den als Bewegungsempfängergestellteile wirkenden Bewegungsgestellteilen wird durch Fixiervorrichtungen (52-61) ermöglicht.

Die Fixierung des Objekts an den Berührungsstellen mit den Bewegungsgestellteilen (5-9) erfolgt in geeigneter Weise nach bekannten Methoden. z. B. durch Bandagen, Kleben, Fixieren und Vakuumkissen, Schrauben, Gießeinbettung.

Die auf der Basisplatte angebrachte bisher beschriebene Anordnung kann durch eine haubenförmige stabile Abdeckung (62) verschlossen werden, die luftdicht befestigt werden kann. In der Basisplatte (2) sind verschiedene Mehrfach-Durchführungen, z. B. an den Stellen 63, 64 und 65 vorgesehen, die zur energiezuführung, zur Meß- und Steuersignal-Zu- und Abführung sowie zur Erzeugung definierter Versuchsbedingungen (Temperatur, Luftzusammensetzung, Feuchtigkeit, Druckregelung, Flüssigkeitszuführung usw.) dienen. Die Basisplatte 2 kann in einer Drehvorrichtung (z. B. Scharnier (66)) mit einer Hubvorrichtung (67) gegenüber der tischförmigen Gestellunterlage in einem Winkel von 0-90 Grad hochgeklappt werden, um die Objekte in Schwerkraft- oder sonstigen Beschleunigungsfeldern oder aber auch im schwerelosen Zustand in verschiedener Weise positionieren zu können.

Die mit dieser Gestellanordnung erzielbaren Vorteile bestehen gegenüber den bisher bekannten Schüttelgestellen und Vibrationserzeugunggeräten darin, daß die Krafteinleitung sehr genau nach Amplitude und Richtung definiert erfolgt und die daraus resultierende Bewegung und Verformung mit der einwirkenden Kraft sehr genau nach Amplitude, Richtung sowie in ihrer Zeitabhängigkeit korreliert werden kann. Dabei können statische und dynamische Kräfte überlagert auf das Objekt einwirken. Darüber hinaus ist es möglich, durch die wahlweise Veränderung der Krafteinkopplung bzw. der aus der krafteinkopplung resultierenden Verformungsreaktionen den Kraftfluß durch das Objekt gezielt zu variieren und die damit verbundenen Reaktionen des Objekts in bezug auf die Speicherung und die Dämpfung von mechanischer Energie sowie im Hinblick auf die Verformung zu untersuchen.

## Abb. 3

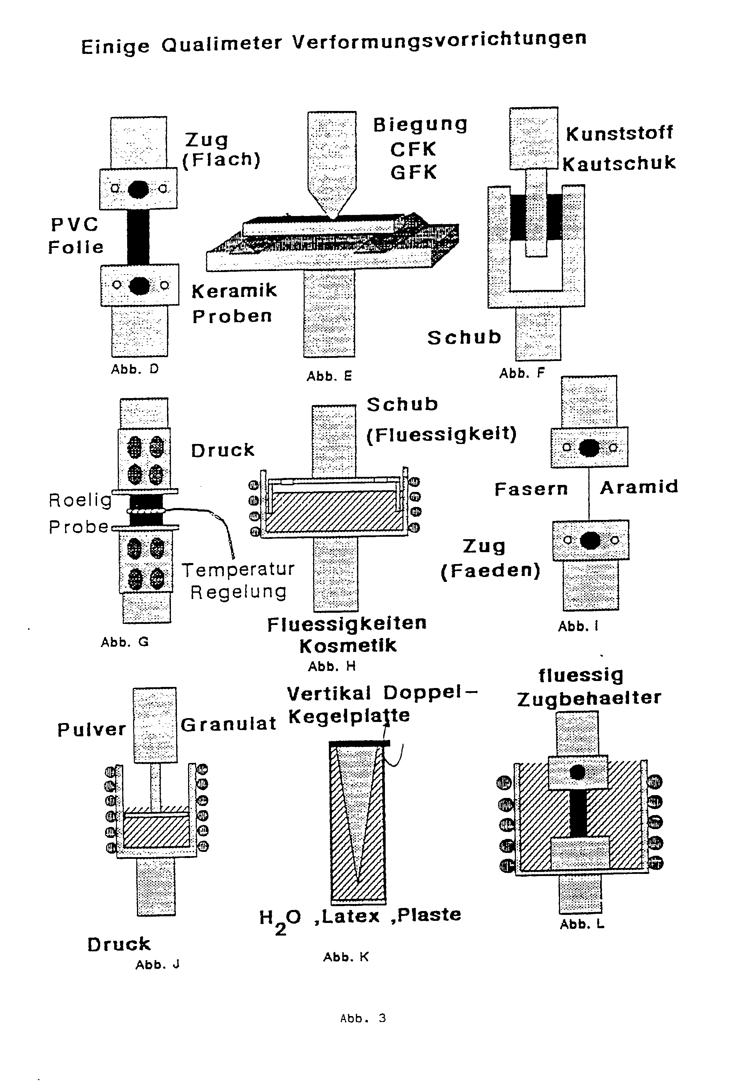

In der Abb. 3 sind eine Reihe von Verformungseinrichtungen dargestellt, und zwar Abb. D, E, F, G, H, I, J, K und Abb. L. Bei diesen auf Abb. 3 dargestellten Verformungsvorrichtungen handelt es sich um bekannte Vorrichtungen zur Untersuchung an nicht lebenden Systemen. Diese bekannten Systeme können in Kombination mit dem neuen System ebenfalls in der neuen Kammer zur Anwendung gebracht werden.

## Abb. 4 und Abb. 5

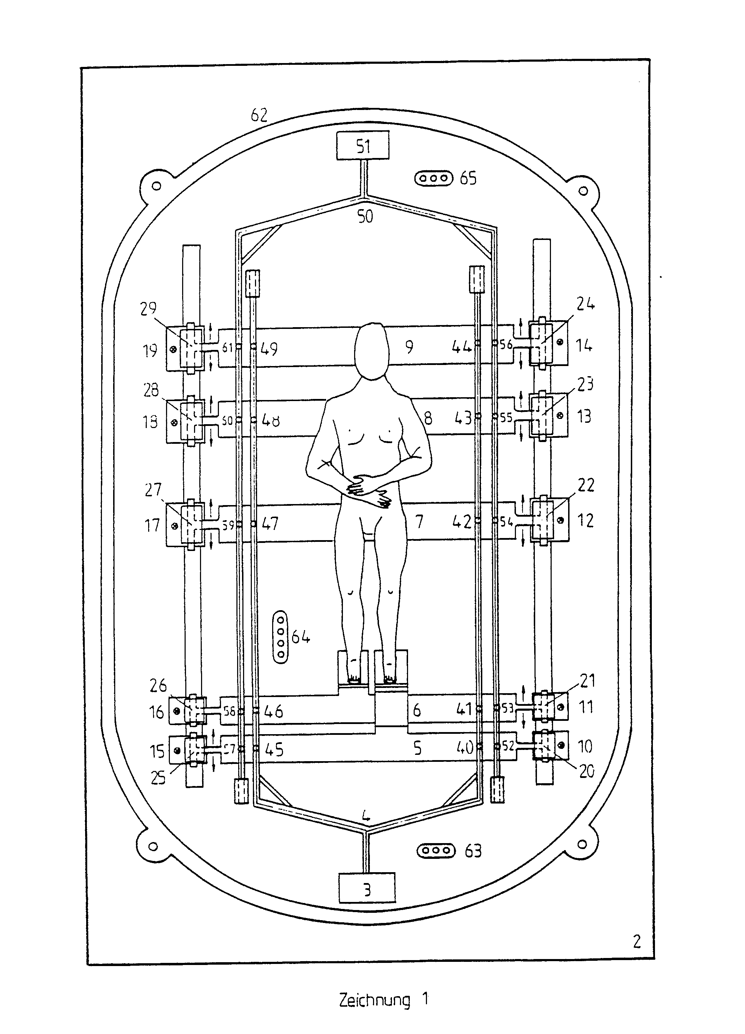

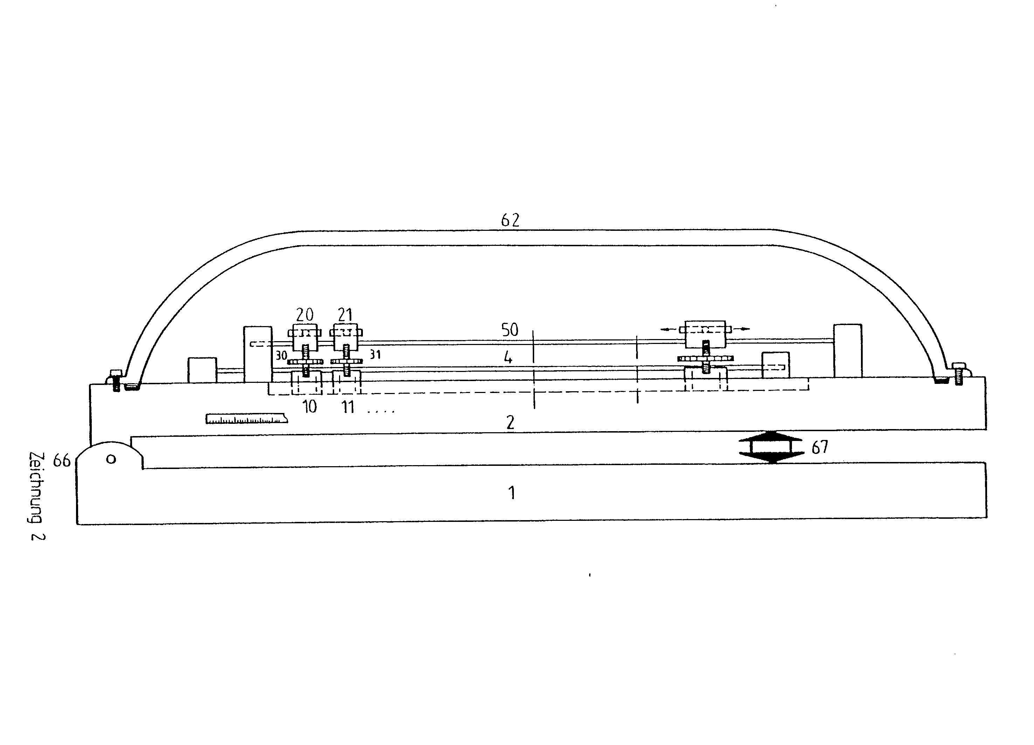

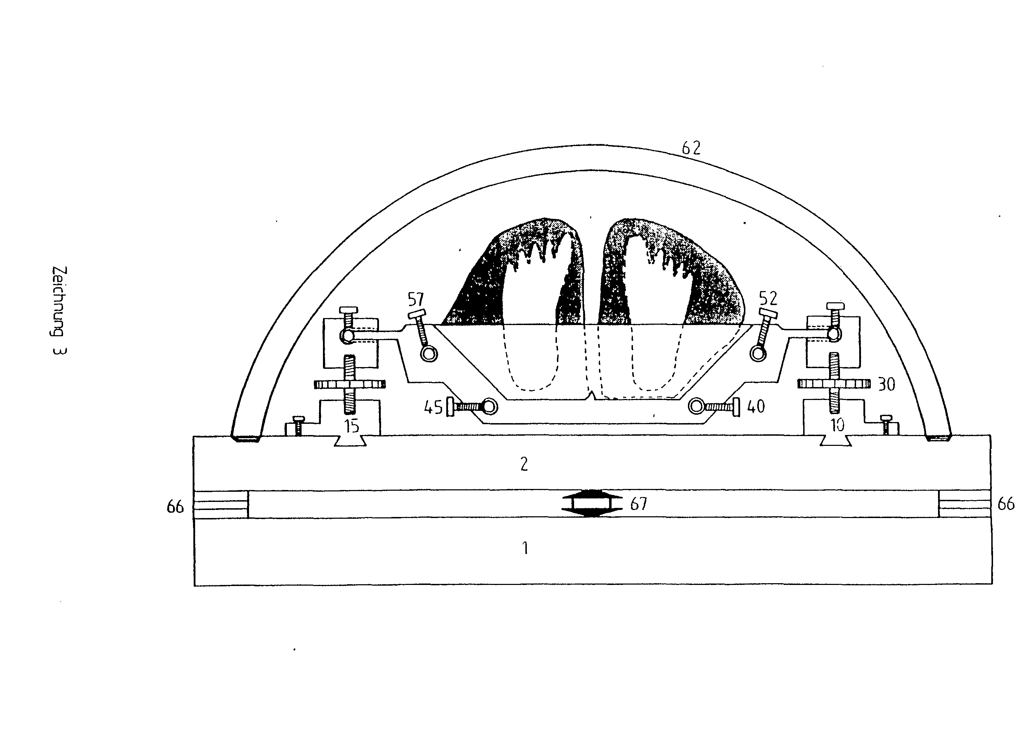

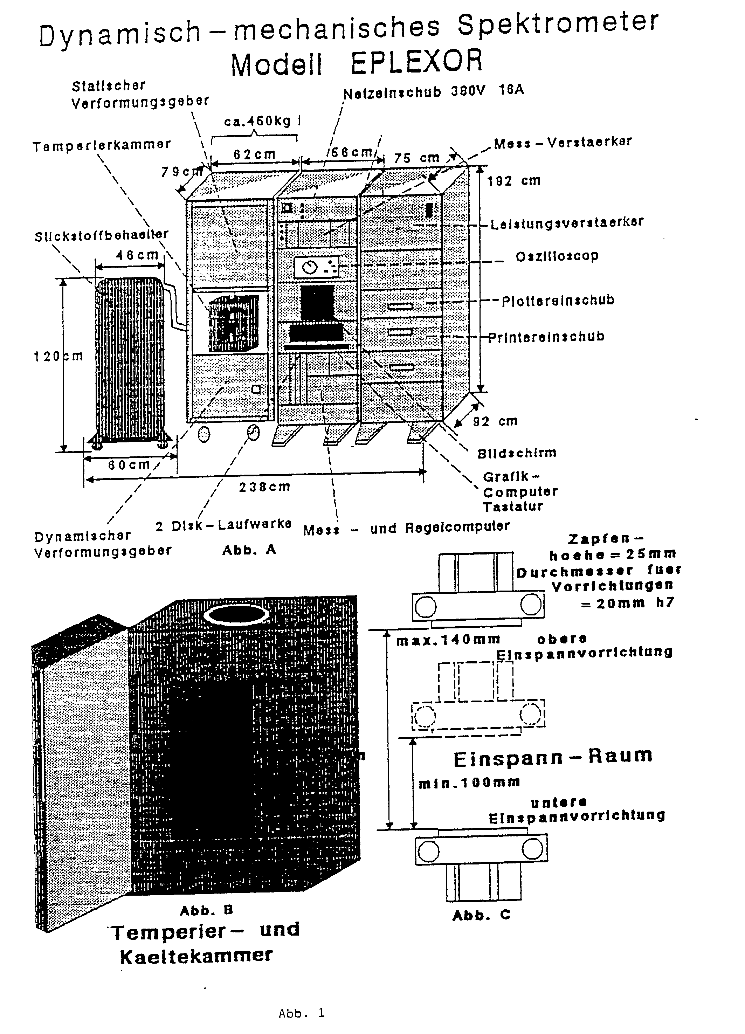

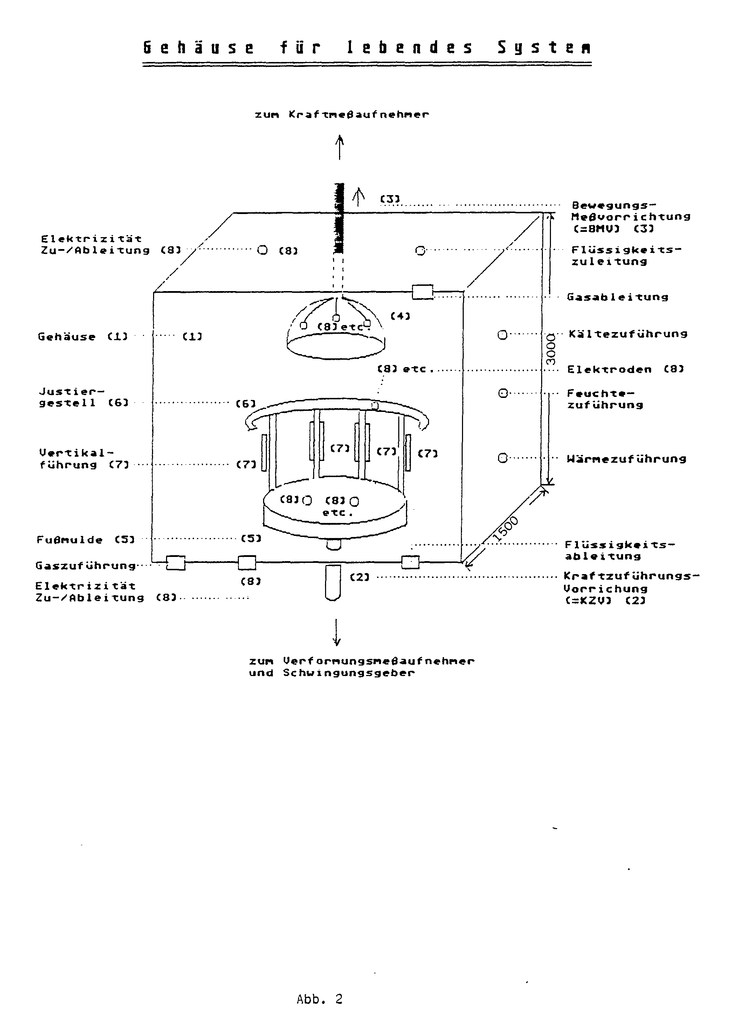

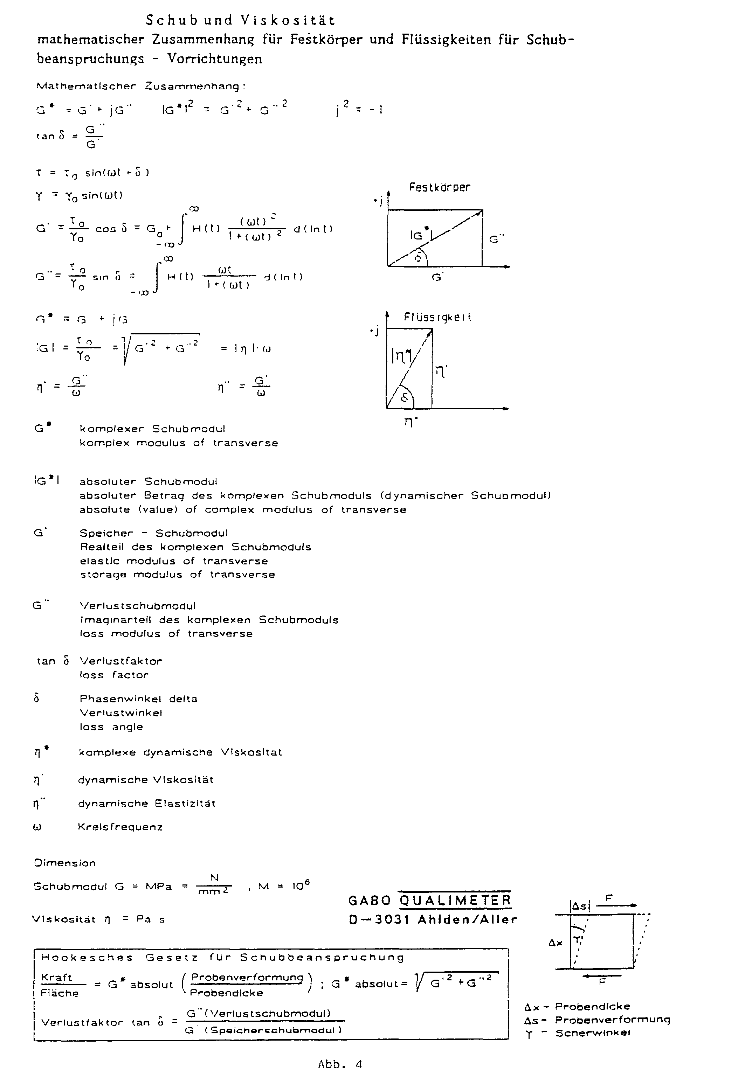

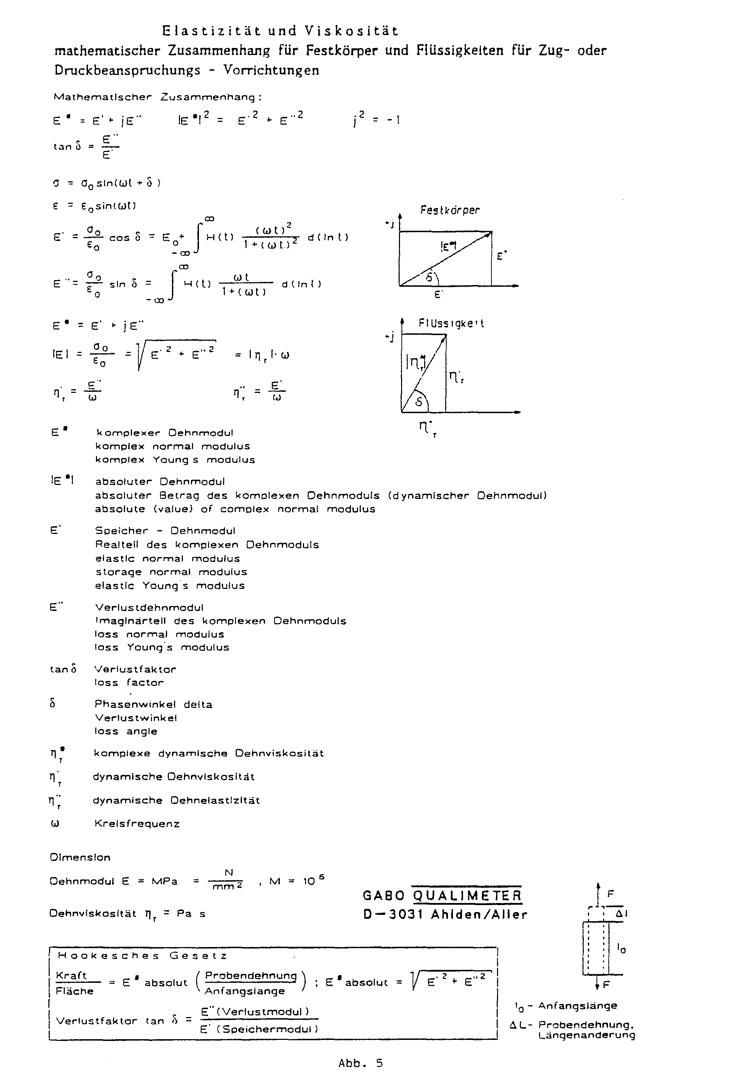

In der Abb. 4 und Abb. 5 sind die mathematischen Zusammenhänge dargestellt, wie sie bisher für Fest körper und Flüssigkeiten gelten und angewandt werden. Erfindungsgemäß werden in Kombination und der in Abb. 2 und den Zeichnungen 2, 3 beschriebenen Vorrichtungen diese mathematischen Zusammenhänge erstmals erfindungsgemäß als neues Verfahren zur Untersuchung am lebenden System zur Anwendung gebracht.

## Ansprüche

1. Verfahren und Vorrichtung zur Messung und Beeinflussung von Eigenschaften eines lebenden Systems, wobei nach der Methode der dynamisch-mechanischen Spektrometrie das lebende System mit verschiedenen konstanten oder nach definierten Funktionen veränderbaren Frequenzen dynamisch, aber auch statisch belastet und entlastet wird und die jeweils bei der entsprechenden Belastung oder Entlastung auftretenden Eigenschaftsänderungen über Kraftmeß- und Verformungsmeßsysteme kontinuierlich verfolgt werden, wobei während oder nach der Messung die zwischen den Kräften und Verformungen auftretenden zeitlichen Phasenverschiebungen mit ausgewertet werden.
Das hierzu benötigte neuartige Verfahren und die Vorrichtung wird in seinem grundsätzlichen Aufbau beispielhaft in Abb. 1, 2, 3, 4 und 5 näher skizziert und es werden nach Anspruch

a.) der zeitliche Verlauf der Eigenschaftsänderungen des lebenden Systems im geschlossenen Kraftfluß kontinuierlich registriert und nach Anspruch

b.) die Eigenschaftsänderungen des lebenden Systems gemäß a.) durch Variationen der statischen und dynamischen Belastung in Verbindung mit der gewünschten Umweltsimulation vom Steuer- und Regelmechanismus erzwungen und nach Anspruch

c.) die Reaktion des lebenden Systems auf die erzwungenen Bedingungen gemäß b.) durch Erfassung der Kräfte, Verformungen, Zeiten, Temperaturen und elektrischen Spannungen und Strömen ausgewertet und beurteilt und nach Anspruch

d.) die neuen Auswertungen der Reaktionen des lebenden Systems gemäß Anspruch a.), b.) und c.) verglichen mit bisher bekannten Methoden wie EKG, EEG oder weiteren.

2. Schüttelvorrichtung nach Anspruch 1, 1a, 1b, 1c, 1d zur Untersuchung des Verhaltens von Objekten gegenüber Krafteinwirkungen, dadurch gekennzeichnet, daß die Vorrichtung aus einem kastenförmigen starren Gestell besteht. In diesem Gestell ist eine unbewegliche Gestellunterlage (1) mit hochklappbarer flacher Basisplatte (2) angeordnet. Auf dieser Platte (2) ist ein Bewegungserzeuger (Kraftgeber) (3) angeordnet. Auf der Platte (2) sind außerdem Bewegungsgestellteile (5-9) in Führungsböcken (10-19) verschiebbar und fixierbar angeordnet. Diese Führungsböcke (10-19) sind auf der Platte (2) in Führungsschienen verschiebbar und auf dieser fixierbar angeordnet.
Die Verbindung zwischen je 2 Führungsböcken und einem zugehörenden Bewegungsgestellteil ist über 2 auf den Führungsböcken angebrachten möglichst reibungsfreien magnetischen oder pneumatischen Lagerungen (20-29) hergestellt, welche mit Justiervorrichtungen (30-39) versehen sind. Einige dieser Bewegungsgestellteile (Auswahl aus 5-9) sind mit dem Schubstangensystem (4) und dem Kraftgeber (3) durch die zugehörigen Fixiervorrichtungen (Auswahl aus 40-49) kraftschlüssig starr verbunden, während die restlichen Bewegungsgestellteile (Rest von 5-9) mit dem Schubstangensystem (50) und dem Bewegungsaufnehmer (51) durch die jeweils zugeordneten Fixiervorrichtungen (Auswahl von 52-61) kraftschlüssig starr verbunden sind.
Der kraftschlüssige Verbund zwischen den mit dem Schubstangensystem (4) verbundenen Bewegungserzeugergestellteilen und den mit dem Schubstangensystem (50) verbundenen Bewegungsempfängergestellteilen ist über das Untersuchungsobjekt hergestellt, welches an den Berührungsstellen mit den Bewegungsgestellteilen in geeigneter herkömmlicher Weise (z. B. durch Schrauben, Kleben, Spannen, Eingießen, Anpressen usw.) möglichst starr befestigt ist.

3. Schüttelvorrichtung nach Anspruch 2, dadurch gekennzeichnet, daß die starre Befestigung des Untersuchungsobjekts auf den Bewegungsgestellteilen (5-9) durch auswechselbare muldenförmige, haubenförmige, ebene oder andere der Form des Untersuchungsobjekts angepaßte Befestigungsteile erfolgt, welche mit Elektroden oder anderen Signal- und Energiezuführungs- und Ableitungsvorrichtungen versehen sind, mit denen dem Untersuchungsobjekt solche Signale und Energien zu- und abgeführt werden.

4. Schüttelvorrichtung nach Anspruch 2 und 3, dadurch gekennzeichnet, daß die Basisplatte (2) Zu- und Abführungen enthält, die zur Versorgung der auf der Basisplatte befindlichen Anordnungsteile mit elektrischer Energie, für eine Gas-, Luft- oder Flüssigkeits-Zu- oder Abführung, zur Zu- und Ableitung von Meß- und Steuersignalen und zur Verbindung mit rechnergesteuerten energiezuführenden und bewegungsmessenden Systemen dienen.

5. Schüttelvorrichtung nach Anspruch 2-4, dadurch gekennzeichnet, daß die gesamte auf der Basisplatte (2) befindliche Anordnung durch einen luftdichten evakuierbaren Kasten oder eine wannenförmige Abdeckungshaube verschließbar ist.

6. Schüttelvorrichtung nach Anspruch 2-5, dadurch gekennzeichnet, daß die Basisplatte in einer Drehvorrichtung mittels eines Scharniers (68) und einer Hubvorrichtung (67) gegenüber der tischförmigen Gestellunterlage (1) in einen Winkel von 0-90 Grad hochklappbar ist.

Zeichnung 1

EP 0 389 996 A1

Zeichnung 2

Zeichnung 3

# Dynamisch–mechanisches Spektrometer
## Modell EPLEXOR

Statischer
Verformungsgeber

Netzeinschub 380V 16A

Temperierkammer

ca.460kg I

Mess – Verstaerker

79cm

62cm

66cm

75 cm

192 cm

Stickstoffbehaelter

46cm

Leistungsverstaerker

Oszilloscop

120cm

Plottereinschub

Printereinschub

92 cm

60cm

Bildschirm

238cm

Grafik–
Computer
Tastatur

Dynamischer
Verformungsgeber

2 Disk–Laufwerke

Mess – und Regelcomputer

Abb. A

Zapfen–
hoehe = 25mm
Durchmesser fuer
Vorrichtungen
= 20mm h7

max.140mm    obere
Einspannvorrichtung

Einspann–Raum

min.100mm

untere
Einspannvorrichtung

Abb. B

Abb. C

Temperier– und
Kaeltekammer

Abb. 1

## Gehäuse für lebendes System

zum Kraftmeßaufnehmer

(3) — Bewegungs-Meßvorrichtung (=BMV) (3)

Elektrizität Zu-/Ableitung (8) — (8) — Flüssigkeits-zuleitung

— Gasableitung

Gehäuse (1) — (1) — (4) — Kältezuführung

(8) etc — Elektroden (8)

(8) etc. — Feuchte-zuführung

Justier-gestell (6) — (6)

Vertikal-führung (7) — (7) — (7) (7) (7) — Wärmezuführung

(8) (8) etc.

Fußmulde (5) — (5) — Flüssigkeits-ableitung

Gaszuführung — (2) — Kraftzuführungs-Vorrichung (=KZV) (2)

Elektrizität Zu-/Ableitung (8)

3000
1500

zum Verformungsmeßaufnehmer und Schwingungsgeber

Abb. 2

# Einige Qualimeter Verformungsvorrichtungen

Zug
(Flach)

PVC
Folie

Keramik
Proben

**Abb. D**

Biegung
CFK
GFK

**Abb. E**

Kunststoff
Kautschuk

Schub

**Abb. F**

Druck

Roelig
Probe

Temperatur
Regelung

**Abb. G**

Schub
(Fluessigkeit)

**Fluessigkeiten
Kosmetik**
**Abb. H**

Fasern    Aramid

Zug
(Faeden)

**Abb. I**

Pulver  Granulat

**Druck**
**Abb. J**

**Vertikal Doppel−
Kegelplatte**

$H_2O$ ,Latex ,Plaste

**Abb. K**

**fluessig
Zugbehaelter**

**Abb. L**

Abb. 3

Schub und Viskosität

mathematischer Zusammenhang für Festkörper und Flüssigkeiten für Schubbeanspruchungs - Vorrichtungen

Mathematischer Zusammenhang:

$$G^* = G' + jG'' \qquad |G^*|^2 = G'^2 + G''^2 \qquad j^2 = -1$$

$$\tan \delta = \frac{G''}{G'}$$

$$\tau = \tau_0 \sin(\omega t + \delta)$$

$$\gamma = \gamma_0 \sin(\omega t)$$

$$G' = \frac{\tau_0}{\gamma_0} \cos \delta = G_0 + \int_{-\infty}^{\infty} H(t) \frac{(\omega t)^2}{1+(\omega t)^2} \, d(\ln t)$$

$$G'' = \frac{\tau_0}{\gamma_0} \sin \delta = \int_{-\infty}^{\infty} H(t) \frac{\omega t}{1+(\omega t)} \, d(\ln t)$$

$$G^* = G + jG$$

$$|G| = \frac{\tau_0}{\gamma_0} = \sqrt[1]{G'^2 + G''^2} = |\eta| \cdot \omega$$

$$\eta' = \frac{G''}{\omega} \qquad\qquad \eta'' = \frac{G'}{\omega}$$

*Festkörper*

*Flüssigkeit*

G*    komplexer Schubmodul
      komplex modulus of transverse

|G*|  absoluter Schubmodul
      absoluter Betrag des komplexen Schubmoduls (dynamischer Schubmodul)
      absolute (value) of complex modulus of transverse

G'    Speicher - Schubmodul
      Realteil des komplexen Schubmoduls
      elastic modulus of transverse
      storage modulus of transverse

G''   Verlustschubmodul
      Imaginärteil des komplexen Schubmoduls
      loss modulus of transverse

tan δ  Verlustfaktor
       loss factor

δ     Phasenwinkel delta
      Verlustwinkel
      loss angle

η*    komplexe dynamische Viskosität

η'    dynamische Viskosität

η''   dynamische Elastizität

ω     Kreisfrequenz

Dimension

Schubmodul $G = MPa = \frac{N}{mm^2}$ , $M = 10^6$

Viskosität $\eta = Pa \; s$

GABO QUALIMETER
D—3031 Ahlden/Aller

Hookesches Gesetz für Schubbeanspruchung

$$\frac{Kraft}{Fläche} = G^* \text{ absolut} \left( \frac{Probenverformung}{Probendicke} \right) ; \; G^* \text{ absolut} = \sqrt{G'^2 + G''^2}$$

$$\text{Verlustfaktor } \tan \delta = \frac{G'' (Verlustschubmodul)}{G' (Speicherschubmodul)}$$

Δx - Probendicke
Δs - Probenverformung
γ - Scherwinkel

Abb. 4

Elastizität und Viskosität

mathematischer Zusammenhang für Festkörper und Flüssigkeiten für Zug- oder Druckbeanspruchungs - Vorrichtungen

Mathematischer Zusammenhang:

$$E^* = E' + jE'' \qquad |E^*|^2 = E'^2 + E''^2 \qquad j^2 = -1$$

$$\tan \delta = \frac{E''}{E'}$$

$$\sigma = \sigma_0 \sin(\omega t + \delta)$$

$$\varepsilon = \varepsilon_0 \sin(\omega t)$$

$$E' = \frac{\sigma_0}{\varepsilon_0} \cos \delta = E_0 + \int_{-\infty}^{\infty} H(t) \frac{(\omega t)^2}{1+(\omega t)^2} \, d(\ln t)$$

$$E'' = \frac{\sigma_0}{\varepsilon_0} \sin \delta = \int_{-\infty}^{\infty} H(t) \frac{\omega t}{1+(\omega t)} \, d(\ln t)$$

$$E^* = E' + jE''$$

$$|E| = \frac{\sigma_0}{\varepsilon_0} = \sqrt{E'^2 + E''^2} = |\eta_r| \cdot \omega$$

$$\eta_r' = \frac{E''}{\omega} \qquad\qquad \eta_r'' = \frac{E'}{\omega}$$

Festkörper

Flüssigkeit

$E^*$    komplexer Dehnmodul
komplex normal modulus
komplex Young's modulus

$|E^*|$    absoluter Dehnmodul
absoluter Betrag des komplexen Dehnmoduls (dynamischer Dehnmodul)
absolute (value) of complex normal modulus

$E'$    Speicher - Dehnmodul
Realteil des komplexen Dehnmoduls
elastic normal modulus
storage normal modulus
elastic Young's modulus

$E''$    Verlustdehnmodul
Imaginärteil des komplexen Dehnmoduls
loss normal modulus
loss Young's modulus

$\tan \delta$    Verlustfaktor
loss factor

$\delta$    Phasenwinkel delta
Verlustwinkel
loss angle

$\eta_r^*$    komplexe dynamische Dehnviskosität

$\eta_r'$    dynamische Dehnviskosität

$\eta_r''$    dynamische Dehnelastizität

$\omega$    Kreisfrequenz

Dimension

Dehnmodul $E$ = MPa $= \frac{N}{mm^2}$ , $M = 10^6$

Dehnviskosität $\eta_r$ = Pa s

GABO QUALIMETER
D—3031 Ahlden/Aller

Hookesches Gesetz

$$\frac{Kraft}{Fläche} = E^* \text{ absolut} \left( \frac{Probendehnung}{Anfangslänge} \right) ; \quad E^* \text{ absolut} = \sqrt{E'^2 + E''^2}$$

$$\text{Verlustfaktor } \tan \delta = \frac{E'' (\text{Verlustmodul})}{E' (\text{Speichermodul})}$$

$l_0$ - Anfangslänge

$\Delta L$ - Probendehnung.
Längenänderung

Abb. 5

**Europäisches Patentamt**

**EUROPÄISCHER TEILRECHERCHENBERICHT,**
der nach Regel 45 des Europäischen Patentübereinkommens für das weitere Verfahren als
europäischer Recherchenbericht gilt

Nummer der Anmeldung

EP 90 10 5607

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | MEDICAL AND BIOLOGICAL ENGINEERING, Band 14, Nr. 3, Mai 1976, Seiten 253-262, Stevenage, GB; G.A. THOMPSON et al.: "In vivo determination of mechanical properties of the human ulna by means of mechanical impedance tests: Experimental results and improved mathematical model" <br><br> * Das ganze Dokument * <br><br> -- | <br><br><br><br><br><br><br><br>1 | A 61 B 5/103 |
| A | JOURNAL OF PHYSICS E SCIENTIFIC INSTRUMENTS, Band 19, Nr. Mai 1986, Seiten 342-346, Ishing, Bristol, GB; P. COLLIER et al.: "An automated dynamic mechanical spectrometer" <br><br> * Das ganze Dokument * <br><br> -- ./. | <br><br><br><br><br><br>1 | RECHERCHIERTE SACHGEBIETE (Int. Cl.4) <br><br> A 61 B <br> G 01 N |

### UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich
ist, auf der Grundlage einiger Patentansprüche sinnvolle Ermittlungen über den Stand der Technik
durchzuführen.

Vollständig recherchierte Patentansprüche: 2-6

Unvollständig recherchierte Patentansprüche: 1

Nicht recherchierte Patentansprüche:

Grund für die Beschränkung der Recherche:

Verfahren zur chirurgischen oder therapeutischen Behandlung des menschlichen oder tierischen Körpers (Siehe Art. 52(4) des Europäischen Patentübereinkommens).

| Recherchenort | Abschlußdatum der Recherche | Prüfer |
|---|---|---|
| Den Haag | 09-07-1990 | FERRIGNO |

**EUROPÄISCHER TEILRECHERCHENBERICHT**

| Kategorie | Kennzeichnung des Dokuments mit Angabe, soweit erforderlich, der maßgeblichen Teile | betrifft Anspruch | KLASSIFIKATION DER ANMELDUNG (Int. Cl. 4) |
|---|---|---|---|
| | **EINSCHLÄGIGE DOKUMENTE** | | |
| A | JOURNAL OF THE ACOUSTICAL SOCIETY OF AMERICA, Band 52, Nr. 2, August 1972, Seiten 577-584, New York, US; T.J. MOORE et al.: "Measurement of specific mechanical impedance of the skin: effects of static force, site of stimulation, area of probe, and presence of a surround" | | |
| | * Das ganze Dokument * | 1 | |
| | -- | | |
| A | EP-A-0 001 127 (INDUSTRIE-AUTO-MATION GmbH & Co.) | | RECHERCHIERTE SACHGEBIETE (Int. Cl. 4) |
| | * Zusammenfassung * | 1 | |
| | -- | | |
| A | US-A-4 195 643 (G.W. PRATT, Jr.) | | |
| | * Das ganze Dokument * | 1,2 | |
| | -- | | |
| A | MEDIZINTECHNIK, Band 24, no. 1, März 1984, Seiten 27-29; Berlin, DD; J. STEINIGER: "Messsystem zur Registrierung des Gesamtenergiestoffwechsels des Menschen" | | |
| | * Das ganze Dokument * | 1,4 | |
| | -- | | |
| A | US-A-4 679 569 (A. ST. J. LEE) | | |
| | * Das ganze Dokument * | 1,3 | |
| | ------------- | | |